# EUROPÄISCHE PATENTSCHRIFT

(11) **EP 1 216 622 B1**
(45) Veröffentlichungstag und Bekanntmachung des Hinweises auf die Patenterteilung: **14.11.2007**
(21) Anmeldenummer: 01128488.2
(22) Anmeldetag: 07.12.2001
(51) Int. Cl.: A23L 1/22, A23C 9/13, G01N 33/50

(54) **Verfahren zur Anpassung von Aromamischungen**
Method of adaptation of aroma mixtures
Méthode d'adaptation de mélange d'arômes

(30) Priorität: 20.12.2000 DE 10063487
(43) Veröffentlichungstag der Anmeldung: 26.06.2002
(73) Patentinhaber: Symrise GmbH & Co. KG, 37603 Holzminden (DE)
(72) Erfinder: Reinders, Gerald, Dr., 33154 Salzkotten (DE); Erfurt, Harry, 37170 Uslar (DE)
(74) Vertreter: Eisenführ, Speiser & Partner

(56) Entgegenhaltungen:
- LELAND J V: "Flavor interactions: the greater whole." FOOD TECHNOLOGY, Bd. 51, Nr. 1, 1997, Seiten 75-80, XP009057460
- WELTY W M ET AL: "Effects of milk fat, cocoa butter, or selected fat replacers on flavor volatiles of chocolate ice cream" JOURNAL OF DAIRY SCIENCE, Bd. 84, Nr. 1, Januar 2001 (2001-01), Seiten 21-30, XP002355006 ISSN: 0022-0302
- LI ZHENG ET AL: "Effects of milk fat content on flavor perception of vanilla ice cream" JOURNAL OF DAIRY SCIENCE, Bd. 80, Nr. 12, Dezember 1997 (1997-12), Seiten 3133-3141, XP002355007 ISSN: 0022-0302

## Beschreibung

Die vorliegende Erfindung betrifft ein Verfahren zur Anpassung von Aromamischungen sowie deren Verwendung zur Veränderung und Optimierung der Aromaeigenschaften von Lebensmitteln.

Das Aroma eines Lebensmittels wird neben den größtenteils nicht flüchtigen Geschmackskomponenten (süß, sauer, salzig, bitter) im wesentlichen durch flüchtige Verbindungen bestimmt. Die geruchsaktiven flüchtigen Verbindungen werden retronasal in der Regio olfocatoria, einem Gewebe im inneren oberen Teil der Nase, an den Riechrezeptoren wahrgenommen (Trends in Food Sience and Technology (1996) Vol. 7, 425-431; Food Technology (1997) 51 (1) 75-80).

Die Zusammensetzung des Atems, der retronasal vom Mundraum zum Riechephitel gelangt, ist somit entscheidend für die Aromawahrnehmung. Die flüchtigen Verbindungen werden während des Kauens im Mund aus dem Lebensmittel freigesetzt. Der Anteil an flüchtigen Verbindungen wird durch Phasenverteilungsprozesse innerhalb des Lebensmittels (Kernst-Verteilung) und zwischen Lebensmittel und Gasphase im Mundraum (Henry-Verteilung) bestimmt. Für den zeitlichen Ablauf der Aromafreisetzung spielen zudem Diffusionsprozesse im Lebensmittel eine entscheidende Rolle (Food Reviews International (1991) 7 (2), 137-184; Flavor Chemistry: 30 years of Progress (1999), 397-405; Fluid Phase Equilibra (1999) 158-160, 657-671; J. Agric. Food Chem. (2000) 48, 1278-1284).

Die Verteilungs- und Diffusionskonstanten sind für jede einzelne Aromaverbindung unterschiedlich, so dass sich die Zusammensetzung des Aromas in der Dampfphase über dem Lebensmittel mit der Zusammensetzung und Struktur des Lebensmittels ändert. Somit muss ein Aroma für jedes Lebensmittel neu entwickelt, d.h. an dieses angepasst werden.

Zum Thema "Interaktionen zwischen Aroma und Lebensmittel" und zum Thema "Freisetzung von Aromen beim Kauen" existieren zahlreiche Veröffentlichungen. Im allgemeinen werden die Einflüsse von Lebensmittelbestandteilen isoliert betrachtet und diskutiert. Als Ziel der wissenschaftlichen Arbeiten wird zwar die Übertragung eines Aromaprofils auf ein Lebensmittel genannt. Es sind aber bisher keine Methoden bekannt, mit denen die Aromen angepasst werden können.

In rein theoretischen Arbeiten (International Journal of Food Science and Technology (1995) 30, 425-436, Journal of Food Science (1997) 62 (4) 653-658 und 664; International Journal of Food Science and Technology (1997) 32, 1-9, J. Agric. Food Chem. (1997) 45, 1883-1890, J. Agric. Food Chem. (1998) 46, 2727-2743) wird zwar seit Jahren ein umfassendes mathematisches Modell zur Aromafreisetzung im Mund entwickelt, auf eine Aromaanpassung an Lebensmittelmatrizes wird aber nicht eingegangen. In der Veröffentlichung von Kris B. de Roos und Kees Wolswinkel, wird ferner ein "Non-equilibrium partion model for predicting flavour release in the mouth" beschrieben (Trends in Flavour Research (1994) 15-32).

Die vorliegende Erfindung hat sich nunmehr die Aufgabe gestellt, ein Verfahren zur Verfügung zu stellen, mit dem ein Aromaprofil von einer Matrix auf eine weitere Matrix übertragen werden kann. Hierbei soll gewährleistet sein, dass die Aromakomposition in Abhängigkeit von Zusammensetzung und Struktur der weiteren Matrix geändert wird und somit eine genaue Anpassung an die geänderten physikalischen Eigenschaften in der weiteren Matrix erreicht wird.

Es wurde ein Verfahren zur Anpassung von Aromamischungen gefunden, das dadurch gekennzeichnet ist, dass
a) eine Basismatrix enthaltend eine Aromamischung mit den gewünschten Eigenschaften ausgewählt oder hergestellt wird,
b) die Aromazusammensetzung und - Konzentration im Gasraum über der Basismatrix analysiert wird,
c) das Aroma in eine weitere Matrix eingebracht wird,
d) die Aromazusammensetzung und - Konzentration im Gasraum über der weiteren Matrix analysiert wird, und
e) anhand der Ergebnisse der Analysen in Schritt b) und d) die Aromazusammensetzung in der weiteren Matrix derart verändert wird, dass die Aromaeigenschaften in der weiteren Matrix sensorisch denen der Basismatrix entsprechen.

Das Prinzip des erfindungsgemäßen Verfahrens beruht darauf, dass man zunächst eine Basis mit einem dazu passenden Aroma auswählt, welches die gewünschten Eigenschaften aufweist. D.h. vor allen Dingen die sensorischen Eigenschaften des Aromas sind zu testen. Bei den Basismatrizes kann es sich um ein künstliches Modelsystem oder ein gängiges Lebensmittel handeln.

Die Zusammensetzung der flüchtigen Bestandteile im Kopfraum über der Basismatrix wird sodann analysiert. Dies erfolgt vorzugsweise im Gleichgewicht durch statische Headspace-Gaschromatographie.

Im Anschluss daran wird das gleiche Aroma in eine weitere Matrix eingearbeitet. Bei dieser Matrix handelt es sich ebenfalls vorzugsweise um ein Lebensmittel, das mit neuen Aromaeigenschaften versehen werden soll. D.h. in dem erfindungsgemäßen Verfahren wird eine Aromamischung erstellt, bei der die Zusammensetzung der flüchtigen Aromabestandteile im Kopfraum über der Matrix der der Basismatrix entspricht.

Die Analysen können erfindungsgemäß mit allen bekannten Methoden durchgeführt werden. Bevorzugt werden die statische Headspace-Gaschromatographie und die Massenspektrometrie.

Erfindungsgemäß besonders bevorzugt ist der Einsatz der statischen Headspace-Gaschromatographie. Hierbei erfolgt eine Auswertung anhand der Peakflächen. Die Peakflächen aller identifizierten Aromabestandteile werden auf die Summe von 100 % normiert (Peakflächenprozentwerte).

Anhand der Ergebnisse der Analysen für die Basismatrix und die weitere Matrix wird die Anpassung der Aromaverbindungen an die weitere Matrix vorgenommen. Dies ist notwendig, da die Verteilungs- und Diffusionskonstanten für jede einzelne Aromaverbindung unterschiedlich sind. Die Folge ist, dass sich die Zusammensetzung des Aromas in der Dampfphase über dem Lebensmittel mit der Zusammensetzung und Struktur des Lebensmittels ändert. Infolgedessen ändern sich die sensorischen Eigenschaften mit einer geänderten Struktur und Zusammensetzung des Lebensmittels. Somit muss für jede Matrix, d.h. für jedes Lebensmittel ein Aroma neu entwickelt werden, d.h. angepasst werden. Es reicht nicht aus, ein Aroma, welches für eine bestimmte Matrix (d.h. Lebensmittel) geeignet ist, einfach in eine weitere Matrix, d.h. ein weiteres Lebensmittel, einzubringen.

Demgemäß ist es erfindungsgemäß erforderlich, anhand der Ergebnisse der Analysen für Basismatrix und weitere Matrix eine Anpassung der Zusammensetzung für die weitere Matrix zu erreichen.

In einer erfindungsgemäß bevorzugten Form werden demgemäß sogenannte Korrekturfaktoren ermittelt. Hierfür eignen sich insbesondere die Ergebnisse der statischen Headspace-Gaschromatographie. Zur Berechnung des Korrekturfaktors für jede einzelne Aromaverbindung wird der Quotient aus dem Peakflächenprozentwert der Aromaverbindung in der Basis mit dem Peakflächenprozentwert der Aromaverbindung in der neuen Matrix gebildet. Aromaverbindungen, die nicht detektiert werden, erhalten einen Korrekturfaktor von 1.

Ferner wird die Zusammensetzung (Masse-Prozent) der Aromamischung über die jeweiligen Molmassen in die Molzahlen der Einzelaromaverbindungen umgerechnet. Anschließend wird jede Molzahl mit dem entsprechenden Korrekturfaktor multipliziert. Diese korrigierten Molenbrüche werden auf einen Summenwert von 1,00 normiert (Molenbrüche der angepassten Aromamischung) und über die jeweiligen Molmassen in Masseverhältnisse umgerechnet. Das angepasste Aroma wird in einem solchen Masseverhältnis gemischt, dass die Gesamtmasse dem ursprünglichen Basisaroma wieder entspricht.

Das so angepasste Aroma wird in die neue Matrix eingearbeitet. Anschließend kann durch statistische Headspace-Gaschromatographie wiederum analysiert werden, um das Ergebnis der Aromaanpassung zu überprüfen. Hierfür können wieder die Peakflächenwerte der einzelnen Aromaverbindungen auf 100 % normiert werden. Die Anpassung kann als erfolgreich angesehen werden, wenn das Headspace-Profil (Peakflächenprozentwerte) mit dem Headspace-Profil der Basismatrix übereinstimmt.

Schließlich lässt sich ein sogenannter Intensitätsfaktor bestimmen. Dieser berechnet sich als Quotient aus der Summe der Peakflächenwerte aller Aromaverbindungen im Kopfraum der Basismatrix und der Summe der Peakflächenwerte aller Aromaverbindungen im Kopfraum der weiteren Matrix. Die Intensität des Aromas wird angepasst, indem die dosierte Menge des Aromas mit dem Intensitätsfaktor multipliziert wird.

Als weitere Überprüfung der Anpassung kann auch eine sensorische Dreiecksprüfung durchgeführt werden.

Mit dem vorliegenden erfindungsgemäßen Verfahren ist es möglich, ein Aromaprofil eines Lebensmittels auf ein anderes Lebensmittel zu übertragen. Voraussetzung ist, dass die Rezeptur des Aromas qualitativ vollständig bekannt ist. Der Gehalt einer jeden einzelnen Aromaverbindung eines Aromas wird durch Verwendung von Korrekturfaktoren angepasst, so dass sich eine völlig neue Aromarezeptur ergibt, die für das neue Produkt maßgeschneidert ist. Die Korrekturfaktoren lassen sich beispielsweise aus der statischen Headspace-Gaschromatographie-Messung ermitteln. Hierbei werden nur die leichten bis - mittelflüchtigen Aromastoffe erfasst, möglicherweise ist daher für den Bereich der höher siedenden Bestandteile eine Feinanpassung durch einen Flavoristen nötig. Neben dem Profil wird auch die Intensität des Aromas angepasst. Das Ergebnis der Anpassung ist sensorisch (beispielsweise durch Verkostung) und analytisch nachprüfbar. Überraschend lässt sich mit diesem erfindungsgemäßen Verfahren eine Aromaanpassung wesentlich schneller und zielgerichteter durchführen als durch eine rein flavoristische-sensorische Bearbeitung.

Im folgenden wird die Erfindung unter Bezugnahme auf Beispiele näher beschrieben.

Als Basismatrix wurde eine saure Zuckerlösung verwendet, die aromatisiert wurde. Die Zusammensetzung der flüchtigen Aromastoffe im Kopfraum über der Zuckerlösung wurde durch statische Headspace-Gaschromatographie analysiert. Insgesamt wurden Basismatrizes mit einem Pfirsicharoma und drei Erdbeeraromen erstellt und analysiert.

Als weitere Matrizes wurden Joghurts ausgewählt, und zwar Vollfett- und Light-Joghurt.

Die Aromen wurden unverändert in die Joghurt-Matrizes eingearbeitet. Anschließend erfolgte jeweils eine Analyse der Zusammensetzung der flüchtigen Aromaverbindungen im Kopfraum über der jeweiligen Matrix durch statische Headspace-Gaschromatographie.

Anhand der Quotienten aus den Peakflächenprozentwerten der Aromaverbindungen im Kopfraum über der sauren Zuckerlösung und den Peakflächenprozentwerten der Aromaverbindungen im Kopfraum über den Joghurt-Matrizes wurden sodann die Korrekturfaktoren berechnet.

Schließlich wurden die Molzahlen der Aromenmischungen berechnet. Diese wurden sodann mit den Korrekturfaktoren multipliziert und anschließend in der Summe auf 1 normiert (Molenbrüche).

Aufgrund der nunmehr bestimmten Molenbrüche wurden über die jeweiligen Molmassen die Masseverhältnisse berechnet. Anschließend wurden entsprechend den Masseverhältnissen neue Aromamischungen hergestellt, die den Gesamtmassen der ursprünglichen Basisaromen entsprachen.

Die so angepassten Aromamischungen wurden in die jeweiligen Joghurt-Matrizes eingearbeitet. Zur Kontrolle wurden wiederum statische Headspace-Gaschromatographie-Messungen durchgeführt. Die Flächenwerte der einzelnen Aromaverbindungen wurden auf 100 % normiert. In allen Fällen waren die Anpassungen erfolgreich, was durch Vergleich der normierten Peakflächen mit denen der sauren Zuckerlösung und entsprechende sensorische Vergleiche belegt wurde.

## Patentansprüche

1. Verfahren zur Anpassung von Aromamischungen **dadurch gekennzeichnet**
a) eine Basismatrix enthaltend ein Aromagemisch mit den gewünschten Eigenschaften ausgewählt oder hergestellt wird,
b) die Aromazusammensetzung und -konzentration im Kopfraum über der Basismatrix analysiert wird,
c) das Aromagemisch in eine weitere Matrix eingebracht wird,
d) die Aromazusammensetzung im Kopfraum über der weiteren Matrix analysiert wird,
e) anhand der Ergebnisse der Schritt b) und d) die Aromazusammensetzung in der weiteren Matrix derart verändert wird, dass die Aromaeigenschaften in der weiteren Matrix denen der Basismatrix entsprechen und
f) die neue Aromamischung entsprechend dem Analysenergebnis hergestellt und sensorisch geprüft wird.

2. Verfahren nach Anspruch 1 **dadurch gekennzeichnet**
a) aus den Peakflächenprozentwerten der Analysen der Basismatrix und der weiteren Matrix ein Korrekturfaktor berechnet wird,
b) die Molzahlen der Einzel-Aromaverbindungen der Aromamischung berechnet werden,
c) aufgrund der in Schritt a) ermittelten Korrekturfaktoren eine Anpassung der Aromamischung an die weitere Matrix vorgenommen wird.

3. Verfahren nach einen der Ansprüche 1 oder 2 **dadurch gekennzeichnet, dass** die flüchtigen Bestandteile der Aromamischung im Kopfraum über der Matrix ermittelt werden.

4. Verfahren nach einem der Ansprüche 1 bis 3 **dadurch gekennzeichnet, dass** die Analysen mittels statischer Headspace-Gaschromatographie durchgeführt werden.

5. Verfahren nach Anspruch 4 **dadurch gekennzeichnet, dass** aus den gaschromatographisch ermittelten Peakflächenwerten für die Aromaverbindungen im Kopfraum über der Basismatrix und für die Aromaverbindungen im Kopfraum über der weiteren Matrix ein Quotient zur Berechnung der Korrekturfaktoren gebildet wird.

6. Verfahren nach einem der Ansprüche 1 bis 5 **dadurch gekennzeichnet, dass** die angepasste neue Aromamischung in die weitere Matrix in einer Konzentration eingebracht wird, dass die Peakflächensumme der Aromaverbindungen im Kopfraum über der Matrix der der Basismatrix entspricht.

7. Verfahren nach einem der Ansprüche 1 bis 6 **dadurch gekennzeichnet, dass** es sich bei den Matrizes um Lebensmittel, Kosmetika und Bedarfsgegenstände handelt.

## Claims

1. Process for the adaptation of flavour mixtures, **characterized in that**
a) a base matrix comprising a flavour mixture having the desired properties is selected or prepared,
b) the flavour composition and flavour concentration in the headspace above the base matrix is analysed,
c) the flavour mixture is introduced into another matrix,
d) the flavour composition in the headspace above the other matrix is analysed, and
e) on the basis of the results in step b) and d) the flavour composition in the other matrix is changed in such a manner that the flavour properties in the other matrix correspond to those of the base matrix and
f) the novel flavour mixture is prepared in accordance with the analytical result and subjected to sensory testing.

2. Process according to claim 1, **characterized in that**
a) a correction factor is calculated from the peak area percentages of the analyses of the base matrix and the other matrix,
b) the numbers of moles of the individual flavour compounds in the flavour mixture are calculated,
c) on the basis of the correction factors determined in step a), the flavour mixture is adapted to the other matrix.

3. Process according to one of claims 1 or 2, **characterized in that** the volatile constituents of the flavour mixture in the headspace above the matrix are determined.

4. Process according to one of claims 1 to 3, **characterized in that** the analyses are carried out by means of static headspace gas chromatography.

5. Process according to claim 4, **characterized in that** the peak area values determined by gas chromatography for the flavour compounds in the headspace over the base matrix and for the flavour compounds in the headspace over the other matrix are used to form a quotient for calculating the correction factors.

6. Process according to one of claims 1 to 5, **characterized in that** the adapted novel flavour mixture is introduced into the other matrix at a concentration such that the peak area total of the flavour compounds in the headspace over the matrix corresponds to that of the base matrix.

7. Process according to one of claims 1 to 6, **characterized in that** the matrices are foods, cosmetics and requisites.

## Revendications

1. Procédé d'ajustement de mélanges d'arômes, **caractérisé en ce que**
a) on choisit ou on prépare une matrice de base contenant un mélange d'arômes ayant les propriétés désirées,
b) on analyse la composition et la concentration des arômes dans l'espace de tête au-dessus de la matrice de base,
c) on introduit le mélange d'arômes dans une autre matrice,
d) on analyse la composition des arômes dans l'espace de tête au-dessus de l'autre matrice,
e) à l'aide des résultats des étapes b) et d), on modifie la composition d'arômes dans l'autre matrice de façon que les propriétés des arômes dans l'autre matrice correspondent à celles de la matrice de base,
f) on prépare le nouveau mélange d'arômes conformément au résultat de l'analyse et on le contrôle sur le plan sensoriel.

2. Procédé selon la revendication 1, **caractérisé en ce que**
a) on calcule un facteur de correction à partir des valeurs en pourcentage des surfaces de pics des analyses de la matrice de base et de l'autre matrice,
b) on calcule les nombres de moles des différents composés d'arômes du mélange d'arômes,
c) on ajuste le mélange d'arômes à l'autre matrice sur la base des facteurs de correction déterminés dans l'étape a).

3. Procédé selon l'une des revendications 1 ou 2, **caractérisé en ce que** l'on détermine les constituants volatils de mélange d'arômes dans l'espace de tête au-dessus de la matrice.

4. Procédé selon l'une des revendications 1 à 3, **caractérisé en ce que** l'on effectue les analyses par chromatographie en phase gazeuse par la technique de l'espace de tête statique.

5. Procédé selon la revendication 4, **caractérisé en ce que**, à partir des valeurs des surfaces de pics déterminées par chromatographie en phase gazeuse pour les composés d'arômes se trouvant dans l'espace de tête au-dessus de la matrice de base et pour les composés d'arômes se trouvant dans l'espace de tête au-dessus de l'autre matrice, on forme un quotient pour le calcul des facteurs de correction.

6. Procédé selon l'une des revendications 1 à 5, **caractérisé en ce que** l'on amène le nouveau mélange d'arômes ajusté dans l'autre matrice à une concentration telle que la somme des surfaces de pics des composés d'arômes dans l'espace de tête au-dessus de la matrice correspond à celle de la matrice de base.

7. Procédé selon l'une des revendications 1 à 6, **caractérisé en ce que** les matrices sont des aliments, des cosmétiques et des objets de première nécessité.
